# EUROPÄISCHE PATENTANMELDUNG

(11) **EP 4 286 528 A1**
(43) Veröffentlichungstag der Anmeldung: **06.12.2023**
(21) Anmeldenummer: 22176713.0
(22) Anmeldetag: 01.06.2022
(51) Int. Cl.: C12Q 1/48, G01N 33/573

(54) **VERFAHREN UND VORRICHTUNG ZUM NACHWEIS VON TRANSGLUTAMINASEN**

(71) Anmelder: Technische Universität Darmstadt, 64289 Darmstadt (DE)
(72) Erfinder: Hoenes, Joachim, 64673 Zwingenberg (DE); Weber, Christian, 22393 Hamburg (DE); Schmitz, Katja, 64846 Groß-Zimmern (DE); Krestan, Natascha, 65207 Wiesbaden (DE); Meckel, Tobias, 64297 Darmstadt (DE)
(74) Vertreter: Fuchs Patentanwälte Partnerschaft mbB

(57) **Zusammenfassung**

Die vorliegende Erfindung betrifft ein Verfahren und eine Vorrichtung zum Nachweis von Transglutaminasen, insbesondere von Blutgerinnungsfaktor XIII (FXIII). Die Erfindung basiert auf einem schnellen, Point-of-Care-tauglichen Testprinzip für Transglutaminasen und ermöglicht eine genaue Bestimmung von FXIII in kurzer Zeit und mit einfacher Handhabung.

## Beschreibung

Die vorliegende Erfindung betrifft ein Verfahren und eine Vorrichtung zum Nachweis von Transglutaminasen, insbesondere von Blutgerinnungsfaktor XIII (FXIII). Die Erfindung basiert auf einem schnellen, Point-of-Care-tauglichen Testprinzip für Transglutaminasen und ermöglicht eine genaue Bestimmung in kurzer Zeit und mit einfacher Handhabung.

### Stand der Technik

Blut im Körper dient als Transport -und Kommunikationssystem und ist für die Aufrechterhaltung der Körperfunktionen unerlässlich. Kommt es zu einer Gefäßverletzung, so ist ein schneller Wundverschluss nötig, um die Blutverluste so gering wie möglich zu halten. Er erfolgt über das optimale Zusammenspiel zwischen Thrombozyten und Gerinnungsfaktoren. Die Hauptsubstanz des Thrombus bilden zu Polymeren zusammengelagerte Fibrinmonomere. Diese Fibrinmonomere entstehen durch die Abspaltung niedermolekularer Peptide von Fibrinogen durch die Serin-Protease Thrombin. Zur weiteren Stabilisierung des gebildeten Fibrinnetzes katalysiert Blutgerinnungsfaktor XIII, auch fibrinstabilisierender Faktor genannt, eine kovalente Quervernetzung der Fibrinmoleküle untereinander. Durch diese weitere Stabilisierung ist der Thrombus vor vorzeitiger Lyse geschützt. Die genannten Vorgänge stellen die letzten Schritte der komplexen Blutgerinnungskaskade dar.

Blutgerinnungsfaktor XIII (auch kurz als "Faktor XIII" oder "FXIII" bezeichnet) ist ein Plasmaprotein mit einem Gewicht von 320 kDa. Er liegt im Blutplasma als inaktives Heterotetramer mit je zwei A- und B-Untereinheiten vor. Das Heterotetramer stellt das Proenzym dar; unter physiologischen Bedingungen wird FXIII durch Thrombin aktiviert. Hierbei werden die N-terminalen Aktivierungspeptide zunächst durch Thrombin abgespalten (FXIII) und das Dissoziationsgleichgewicht wird hin zu den Monomeren verschoben. Geringe Calcium-Konzentrationen reichen dann zur Aktivierung aus (FXIIIₐ). Auch eine nicht-physiologische, von Thrombin unabhängige Aktivierung durch hohe Calcium-Konzentrationen (> 100 mM) kann den Komplex zu aktiven Monomeren dissoziieren (FXIII⁰). Aktivierter FXIII verknüpft als Transglutaminase Proteine durch Bildung einer Isopeptidbindung zwischen einem γ-Glutaminrest und dem ε-Aminorest in einer exponierten Lysin-Seitenkette. Bei dieser Reaktion wird Ammoniak frei. In der vorliegenden Offenbarung wird zur Bezeichnung von Aminosäuren oder Aminosäureresten auch der gebräuchliche Dreibuchstabencode verwendet, also beispielsweise "Gln" für Glutamin oder "Lys" für Lysin.

Ein Mangel an FXIII kann zu schweren Blutungen, verzögerter Wundheilung, aber auch zu vermehrten Aborten bei Frauen führen. Erblich bedingter FXIII-Mangel wird jedoch meist bereits im Säuglingsalter aufgrund von Nabelstumpfblutungen diagnostiziert. Diese erbliche Form des FXIII-Mangels ist sehr selten. Ein erworbener Mangel tritt häufiger auf und wird beispielsweise durch eine eingeschränkte Synthesekapazität der Leber, chronisch entzündliche DarmErkrankungen, Sepsis oder Verbrauchskoagulopathien ausgelöst. Zum Nachweis eines FXIII-Mangels eigenen sich konventionelle Gerinnungstests wie Thromboplastinzeit oder aktivierte partielle Prothrombinzeit (aPTT) nicht. Thrombozytenzahl und Fibrinogenkonzentration machen ebenfalls keine Aussage über den FXIII.

Besonders bei schweren Blutungen, z.B. nach Unfällen oder bei größeren Operationen führt ein Mangel an Gerinnungsfaktoren zu Komplikationen wie z.B. erhöhtem Blutverlust. Die Gabe von Vollblut wird heutzutage mehr und mehr unterlassen, da sie zwar Verluste substituiert, aber auch neue Ungleichgewichte zwischen den Faktoren erzeugt und zusätzliche Komplikationen verursachen kann. Stattdessen versucht man, durch gezielte Gabe von einzelnen Faktoren das physiologische Gleichgewicht wiederherzustellen. Entsprechende Präparate sind für Fibrinogen und auch für FXIII verfügbar.

Derzeit kann die Aktivität von FXIII im klinisch-chemischen Labor z.B. photometrisch bestimmt werden. Dazu wird FXIII der Probe durch Thrombin aktiviert und verknüpft ein Glutamin-Donor-Substrat mit einem Amin-Donor, z.B. Glycin-Ethylester, unter Freisetzung von Ammoniak. Der so entstandene Ammoniak kann in einer gekoppelten enzymatischen Reaktion bestimmt werden. NADH, α-Ketoglutarat und freier Ammoniak werden von der Glutamatdehydrogenase zu NAD⁺ und Glutamat umgewandelt. Die Abnahme von NADH wird über die Extinktion bei 340 nm gemessen und ist direkt proportional zur FXIII-Aktivität.

Alternativ kann Faktor XIII durch Antikörper in einem Enzyme-Linked Immunosorbent Assay (ELISA) nachgewiesen werden. Faktor XIII bindet dabei an einen spezifischen Antikörper, der im Testgefäß immobilisiert ist. Nach Auswaschen anderer Substanzen bindet ein zweiter Antikörper an den immobilisierten Faktor XIII. Ein an den Antikörper konjugiertes Enzym setzt das anschließend zugegebene signalgebende Substrat um. Die gebildete Substanz kann photometrisch oder fluorimetrisch quantifiziert und daraus der FXIII-Spiegel bestimmt werden.

Beide Tests erfordern einen Transport der Probe ins klinisch-chemische Labor, eine Zentrifugation zur Plasmagewinnung, die Durchführung des eigentlichen Tests sowie eine Auswertung und Rückmeldung an den Auftraggeber. Solche Abläufe unter Einbeziehung des klinischen Labors sind (auch bei anderen Testverfahren) in akuten Fällen zu langsam, um die Notwendigkeit einer Gabe zu beurteilen und die korrekte Dosierung der Präparate zu bestimmen. Alternative Testverfahren sind Thromboelastometrie (eher qualitativ und recht störanfällig) sowie Enzymimmunoassays (sehr aufwändig und teuer, keine Aktivitäts- sondern Proteinbestimmung).

Point of Care (POC) Schnelltests könnten vor Ort (z.B. im Operationssaal) angewandt werden und eine korrekte Entscheidung innerhalb weniger Minuten ermöglichen, sind jedoch bislang aus einer Vielzahl von Gründen nicht verfügbar.

Blut ist ein hochkomplexes Gemisch aus Zellen (Hauptbestandteil Erythrozyten) und im Serum gelösten Bestandteilen, wie Proteinen und Lipiden. Die starke Lichtabsorption im Hämoglobin macht photometrische oder fluorimetrische Bestimmungen in Vollblut schwierig bis unmöglich. Elektrochemische Bestimmungen werden von den Zellen und von der Viskosität der Blutmatrix ebenfalls stark gestört. Eine (teilweise) Korrektur ist mit Verfahren wie der Impedanzspektroskopie möglich, jedoch ebenfalls mit großem Aufwand verbunden.

Eine schnelle Abtrennung der Erythrozyten ist durch Glasfaservliese möglich. Lösliche Blutbestandteile bleiben allerdings im so gewonnenen Blutplasma. Speziell die hohen Konzentrationen von Serum-Albumin und Fibrinogen haben das Potential, Tests für FXIII zu stören. Bekannt ist z.B. der Dansylcadaverin-Test, bei dem ein fluoreszenzmarkiertes Amin in ein glutaminhaltiges Protein, z.B. Dimethyl-Casein, eingebaut wird. Die Blauverschiebung und Verstärkung der Fluoreszenz durch die veränderte chemische Umgebung des Fluorophors kann als Maß für die Aktivität von FXIII herangezogen werden. Serum-Albumin in physiologischen Konzentrationen bindet allerdings das Dansylcadaverin, entzieht es der enzymatischen Reaktion und verschiebt das Fluoreszenzspektrum vorab, so dass die eventuell noch stattfindende Restreaktion nicht mehr sichtbar wird.

Fibrinogen ist eine weitere Störkomponente: Die physiologische Aktivierung von FXIII erfordert Spaltung durch Thrombin; alternativ kann mit hohen Ca²⁺-Konzentrationen gearbeitet werden. In beiden Fällen wird die Gerinnungsreaktion angestoßen; das Gerinnsel behindert durch Lichtstreuung und hohe Viskosität die Nachweisreaktionen.

Die einzige bekannte Methode zur Bestimmung von FXIII aus Vollblut ist die Thromboelastografie (EP 2 474 830 A1). In späteren Phasen der Gerinnung (also nach längerer Beobachtungszeit) sorgt FXIII für eine Verfestigung des Gerinnsels, deren Geschwindigkeit und Ausmaß vom Gehalt der Probe an FXIII abhängen. Das Verfahren ist angesichts der längeren Beobachtungszeit verhältnismäßig langsam. Außerdem beeinflussen viele andere Inhaltsstoffe des Blutes (Zellen, Fibrinogen, Serumalbumin, das Blut verdünnende Agenzien) und die einsetzende Fibrinolyse die Ergebnisse, so dass keine genauen FXIII-Bestimmungen möglich sind.

Als Ergebnis dieser Probleme bietet der Stand der Technik kein Verfahren, mit dem eine genaue Bestimmung von Transglutaminasen, insbesondere von FXIII in angemessen kurzer Zeit und mit einfacher Handhabung möglich wäre. Aufgabe der vorliegenden Erfindung ist vor diesem Hintergrund die Bereitstellung eines schnellen, POC-tauglichen Testprinzips aus humanem Blut.

### Beschreibung der Erfindung

Das Verfahren der vorliegenden Erfindung eignet sich für alle Transglutaminasen. Die Bestimmung der enzymatischen Aktivität von Blutgerinnungsfaktor XIII (FXIII) in einer Probe ist besonders bevorzugt.

Die Bindung erfolgt an einen immobilisierten Glutamindonor. Dieser kann gleichzeitig das zum Nachweis verwendete Substrat sein, muss es aber nicht. Zum Nachweis der Aktivität kann zusätzliches Substrat hinzugegeben werden, entweder der gleiche Glutamindonor oder ein anderer.

Der Nachweis kann entweder über Auswaschen von ungebundenem Amindonor und Nachweis des im Reaktivfeld verbleibenden erfolgen oder bevorzugt über eine Spektralverschiebung des Chromophors am Amindonor durch die Bindung. Dafür kann eine Reihe von Fluorophorresten eingesetzt werden, die auf die geänderte Polarität der Umgebung direkt reagieren, z.B. Dansyl, Dapoxyl, 4-Chlor-7-nitrobenzofurazan (NBD), 6-Dimethylamino-2,3-naphthalimid. Solche Fluorophore können unter dem Begriff "spektroskopische Polaritätssensoren" zusammengefasst werden. Die "Menge" an eingebautem Amin kann insbesondere indirekt spektroskopisch bestimmt werden; die Aktivität der Transglutaminase kann insbesondere über die zeitliche Änderung des spektroskopischen Signals bestimmt werden.

Als Linker zwischen Chromophor und Glutamin eignen sich insbesondere Diamine, bevorzugt Putrescin, Cadaverin und/oder Carbohydrazid.

In einem ersten Aspekt betrifft die Erfindung ein Verfahren zur Bestimmung der enzymatischen Aktivität einer Transglutaminase, insbesondere von Blutgerinnungsfaktor XIII (FXIII), in einer Probe, wobei das Verfahren die folgenden Schritte umfasst:
a) Bereitstellen eines immobilisierten Glutamindonors, z.B. eines FXIII-Substrats, wobei der Glutamindonor ein Peptid ist, insbesondere ein Polypeptid oder Protein, und wobei der Glutamindonor mindestens einen Glutamin-Rest umfasst,
b) Aktivierung der in der Probe vorhandenen Transglutaminase,
c) Inkontaktbringen der Probe mit dem Glutamindonor, so dass in der Probe vorhandene Transglutaminase an den Glutamindonor bindet,
d) Entfernung nicht an den Glutamindonor gebundener Bestandteile der Probe,
e) Inkontaktbringen der an den Glutamindonor gebundenen Transglutaminase mit einem Amindonor, so dass der Amindonor von der Transglutaminase an ein Glutamindonorsubstrat gebunden wird, und
f) Bestimmung der enzymatischen Aktivität der Transglutaminase anhand eines Signals, das durch die Bindung des Amindonors an das Glutamindonorsubstrat erzeugt wird.

Bevorzugt ist das Signal proportional zur Geschwindigkeit der von der Transglutaminase katalysierten Bindung des Amindonors an das Glutamindonorsubstrat.

Die Schritte werden insbesondere in der angegebenen Reihenfolge a) bis f) durchgeführt.

Der immobilisierte Glutamindonor ist bevorzugt das Glutamindonorsubstrat, an das der Amindonor durch die Glutaminase gebunden wird.

Die Transglutaminase ist bevorzugt ausgewählt aus der Gruppe der humanen Transglutaminasen. Besonders bevorzugt ist die Transglutaminase Blutgerinnungsfaktor XIII (FXIII).

Bevorzugt enthält die Probe genau eine Transglutaminase.

Die Probe ist bevorzugt eine Blutprobe, insbesondere eine humane Blutprobe. In anderen Ausführungsformen kann die Probe auch eine Gewebeprobe sein, beispielsweise ein Extrakt aus einem Gewebe.

Die Probe enthält Transglutaminase, insbesondere FXIII, bevorzugt in einer Aktivität von 5% bis 200%, beispielsweise von 25% bis 175%, von 50% bis 150%, von 75% bis 125%, oder von 90% bis 110% bezogen auf die Aktivität in Standardplasma. Die Aktivität beträgt bevorzugt mindestens 5%, mindestens 25%, mindestens 50%, mindestens 75%, oder mindestens 90% bezogen auf die Aktivität in Standardplasma. Die Aktivität beträgt bevorzugt höchstens 200%, höchstens 175%, höchstens 150%, höchstens 125%, oder höchstens 110% bezogen auf die Aktivität in Standardplasma.

Gemäß Schritt a) des erfindungsgemäßen Verfahrens wird ein immobilisierter Glutamindonor, insbesondere ein immobilisiertes Glutamindonorsubstrat, bevorzugt ein immobilisiertes FXIII-Substrat bereitgestellt. Das (FXIII-)Substrat kann ein physiologisches (FXIII-)Substrat sein. In besonders bevorzugten Ausführungsformen ist das (FXIII-)Substrat kein physiologisches (FXIII-)Substrat. Mit (FXIII-)Substrat ist insbesondere gemeint, dass die Transglutaminase (insbesondere FXIII) die Reaktion eines Glutamin-Rests des Substrats mit der Aminogruppe eines Amindonors katalysieren kann. Wenn der Glutamindonor zwar mindestens einen Glutamin-Rest umfasst, die Transglutaminase (insbesondere FXIII) jedoch keine Bindung zwischen Glutamin-Rest und Amindonor katalysieren kann, ist der Glutamindonor kein (FXIII)Substrat.

Der Glutamindonor kann insbesondere ein Peptid sein, bevorzugt ein Polypeptid oder Protein. Der Glutamindonor enthält mindestens einen Glutamin-Rest. Bevorzugt enthält der Glutamindonor mehrere Glutamin-Reste, beispielsweise mindestens zwei, mindestens drei, oder mindestens fünf Glutamin-Reste. Bevorzugt sind Proteine mit einem Molgewicht von 10 bis 300 kDa, beispielsweise von 10 bis 100 kDa oder von 12 bis 30 kDa. Das Molgewicht des Glutamindonors kann beispielsweise mindestens 10 kDa, oder mindestens 12 kDa betragen. Das Molgewicht des Glutamindonors kann beispielsweise höchstens 300 kDa, höchstens 100 kDa oder höchstens 30 kDa betragen. Bevorzugt liegt der Glutamingehalt des Glutamindonors in einem Bereich von 3% bis 15% bezogen auf die Gesamtanzahl an Resten, beispielsweise in einem Bereich von 5% bis 12% oder von 7% bis 10%. Der Glutamingehalt des Glutamindonors kann beispielsweise mindestens 3%, mindestens 5% oder mindestens 7% betragen. Der Glutamingehalt des Glutamindonors kann beispielsweise höchstens 15%, höchstens 12% oder höchstens 10% betragen.

Besonders bevorzugt ist der Glutamindonor ein Substrat der Transglutaminase, auch Glutamindonorsubstrat genannt. Dies hat den Vorteil, dass der immobilisierte Glutamindonor als Glutamindonorsubstrat fungieren kann, an das die Transglutaminase den Amindonor bindet. Dadurch kann auf die Zugabe von zusätzlichem Substrat der Transglutaminase verzichtet werden. Es sind jedoch auch Ausführungsformen umfasst, in denen der immobilisierte Glutamindonor nicht mit dem Glutamindonorsubstrat des Schrittes e) des Verfahrens der vorliegenden Erfindung identisch ist. Insbesondere in solchen Ausführungsformen wird zusätzlich zum immobilisierten Glutamindonor noch Glutamindonorsubstrat zugegeben, an das der Amindonor durch die Transglutaminase gebunden werden kann. Es ist beispielsweise möglich, vor Durchführung des Schrittes e) eine Mischung oder Lösung bereitzustellen, die Amindonor und Glutamindonorsubstrat umfasst, und diese in Schritt e) mit der an den Glutamindonor gebundenen Transglutaminase in Kontakt zu bringen, so dass die Bindung zwischen Amindonor und Glutamindonorsubstrat durch die Transglutaminase gebildet werden kann. Die Zugabe von Glutamindonorsubstrat kann auch dann erfolgen, wenn der immobilisierte Glutamindonor selbst bereits ein Glutamindonorsubstrat ist.

In der vorliegenden Offenbarung beschreiben die Begriffe "Peptid" oder "Peptide" zusammenfassend Moleküle, die Aminosäuren enthalten, die über Peptidbindungen miteinander verknüpft sind. Dies beinhaltet Oligopeptide (Peptide mit bis zu zehn Aminosäureresten) und Polypeptide (Peptide mit mehr als zehn Aminosäureresten). Polypeptide mit mehr 50 Aminosäureresten werden auch als Proteine bezeichnet. Die Begriffe "Peptid" oder "Peptide", wie in der vorliegenden Offenbarung verwendet, umfassen ausdrücklich auch Proteine.

Gemäß Schritt a) des Verfahrens liegt der Glutamindonor, insbesondere das FXIII-Substrat immobilisiert vor. Die Immobilisierung beinhaltet insbesondere die Bindung des Glutamindonors an einen Träger, bevorzugt Sepharose, Cellulose oder Papier, jeweils bevorzugt funktionalisiert mit DEAE (Diethylaminoethyl-Gruppe) oder anderen kationischen Gruppen. Die Immobilisierung kann zusätzlich oder alternativ auch eine Fällung des Glutamindonors, insbesondere des FXIII-Substrats in Gegenwart des Trägers beinhalten, beispielsweise eine Fällung mit Ca²⁺.

Der immobilisierte Glutamindonor, insbesondere der Substrat-Träger-Komplex, kann in verschiedenen Formen vorliegen. Beispielsweise ist es möglich, dass der immobilisierte Glutamindonor in Form einer Suspension vorliegt, insbesondere in einem Reaktionsgefäß. Es ist auch möglich, den Glutamindonor auf einem Festkörper zu immobilisieren, beispielsweise auf einem Papierträger in einem Teststreifen.

Die Immobilisierung des Glutamindonors umfasst bevorzugt eine oder mehrere der folgenden Maßnahmen:
- Fällung und Adsorption
- Ionische Bindung an DEAE-funktionalisierte Oberflächen
- Kovalente Bindung durch Formaldehyd, Glutardialdehyd
- Kovalente Bindung unter Verwendung von EDC/NHS bei Amin- oder COOHfunktionalisierter Trägeroberfläche
- Kovalente Bindung durch Dimethylsuberimidat, Dimethylpimelimidat, Dimethyladipimidat bei aminfunktionalisierter Trägeroberfläche
- Kovalente Bindung durch Trägergebundene Epoxide, Isocyanate
- Kovalente Bindung über Aminogruppen an BrCN-aktivierte Polysaccharide.

Der immobilisierte Glutamindonor kann Modifikationen, beispielsweise chemische Modifikationen aufweisen. Besonders bevorzugt sind Modifikationen von Lysin-Resten. Insbesondere kann der Glutamindonor methyliert, acetyliert oder succinyliert sein, beispielsweise dimethyliert, insbesondere N,N-dimethyliert oder auch acetamidiniert.

Insbesondere kann der Glutamindonor methyliert sein, beispielsweise dimethyliert, insbesondere N,N-dimethyliert. Die Dimethylierung erfolgt insbesondere durch Umsetzung des Glutamindonors mit Formaldehyd und Natriumborhydrid. Alternativ oder zusätzlich können ein oder mehrere andere Reduktionsmittel eingesetzt werden. Die Reaktion richtet sich nach der Zugänglichkeit der Lysine - gut zugänglich reagiert am schnellsten. Der Dimethylierungsgrad wird durch die Menge der zugegebenen Reagenzien und die Reaktionsdauer gesteuert. Ein möglichst hoher Dimethylierungsgrad der frei zugänglichen Lysine ist bevorzugt, weil das die Quervernetzung innerhalb eines Proteins oder zwischen den Proteinen verhindert. Bevorzugt beträgt der Dimethylierungsgrad mehr als 90%.

Zusätzlich oder alternativ zur Methylierung kann der immobilisierte Glutamindonor bevorzugt succinyliert oder acetamidiniert sein.

Besonders bevorzugt ist der Glutamindonor ausgewählt aus der Gruppe bestehend aus Casein, insbesondere Dimethylcasein, und Lysozym, insbesondere succinyliertes Lysozym, bevorzugt succinyliertes Hühnereiweißlysozym (*Gallus gallus*)*.* Casein aus Kuhmilch (*Bos taurus*) ist bevorzugt. Dimethylcasein ist dem Fachmann bestens bekannt, da es üblicherweise zur Proteasebestimmung über den Nachweis neu gebildeter endständiger Aminogruppen verwendet wird.

Gemäß Schritt b) des erfindungsgemäßen Verfahrens wird die in der Probe vorhandene Transglutaminase aktiviert. In Ausführungsformen, in denen die Transglutaminase FXIII ist, kann die Aktivierung durch Thrombin erfolgen. Allerdings sollte in diesem Fall beispielsweise durch Zusatz von Hemmstoffen der Fibrinaggregation, z.B. durch das Tetrapeptid GPRP, die Gerinnung vermieden werden.

Bevorzugt werden hohe Ca²⁺-Konzentrationen zur Aktivierung verwendet. Dies gilt insbesondere, wenn die Transglutaminase FXIII ist, aber auch bei anderen Transglutaminasen. Bei einer Aktivierung mit Ca²⁺ liegt die Ca²⁺-Konzentration bevorzugt in einem Bereich von 100 bis 500 mM, beispielsweise von 200 bis 400 mM. Die Ca²⁺-Konzentration kann beispielsweise mindestens 100 mM oder mindestens 200 mM betragen. Die Ca²⁺-Konzentration kann beispielsweise höchstens 500 mM oder höchstens 400 mM betragen. Auch niedrigere Ca²⁺-Konzentrationen, beispielsweise Konzentrationen von 1 bis <100 mM, insbesondere von 1 bis 10 mM sind in Verbindung mit hoher lonenstärke möglich. Eine hohe lonenstärke kann beispielsweise durch NaCI-Konzentration in einem Bereich von 200 bis 1000 mM, insbesondere von 400 bis 700 mM erhalten werden.

In Ausführungsformen, in denen die Transglutaminase FXIII ist, liegt FXIII nach der Aktivierung gemäß Schritt b) bevorzugt als FXIIIₐ (durch Thrombin gespalten) oder als FXIII⁰ (durch Ca²⁺-lonen aktiviert) vor.

Gemäß Schritt c) des erfindungsgemäßen Verfahrens wird die Probe mit dem immobilisierten Glutamindonor in Kontakt gebracht. Die Einzelheiten des Inkontaktbringens richten sich im Wesentlichen nach der Art der Immobilisierung des Glutamindonors. Wenn der immobilisierte Glutamindonor als Suspension vorliegt, insbesondere in einem Reaktionsgefäß, kann das Inkontaktbringen beispielsweise dadurch erfolgen, dass die Probe zu der Suspension hinzugegeben wird, beispielsweise durch Zugabe der Probe in das Reaktionsgefäß hinzugegeben wird. Wenn der Glutamindonor hingegen immobilisiert auf einem Festkörper, wie beispielsweise einem Teststreifen, vorliegt, kann das Inkontaktbringen erfolgen, indem die Probe und der Festkörper, insbesondere der Teststreifen, in Kontakt gebracht werden. Die Probe kann beispielsweise an der Stelle auf den Festkörper aufgebracht werden, an der auch der Glutamindonor immobilisiert ist. Es ist jedoch auch möglich, die Probe an einer Stelle aufzubringen, an der nicht der Glutamindonor immobilisiert ist. Dies kann insbesondere dann eine bevorzugte Möglichkeit sein, wenn der Festkörper, insbesondere der Teststreifen, derart ausgestaltet ist, dass die Probe, beispielsweise durch Kapillarkräfte, durch den Festkörper wandern und so mit dem immobilisierten Glutamindonor in Kontakt gebracht werden kann.

Es ist möglich, die Probe vor dem Inkontaktbringen mit dem immobilisierten Glutamindonor einem Vorbehandlungsschritt, beispielsweise einem Reinigungsschritt, zu unterziehen. Bevorzugt enthält das Verfahren jedoch keinen Schritt der Vorbehandlung der Probe. Es ist ein besonderer Vorteil der vorliegenden Erfindung, dass die Probe keiner Vorbehandlung bedarf. So ist es insbesondere möglich, die Probe, beispielsweise eine (humane) Blutprobe, nach ihrer Entnahme ohne Vorbehandlung mit dem immobilisierten Glutamindonor in Kontakt zu bringen. Calcium kann zugesetzt werden. Im Falle einer Fällung des Glutamindonors mit Ca²⁺ kann auch das Calcium im gefällten Glutamindonor zur Aktivierung der Transglutaminase beitragen oder die Aktivierung bewirken.

Das Inkontaktbringen gemäß Schritt c) des Verfahrens kann einen Inkubationsschritt umfassen. Der Inkubationsschritt ist besonders vorteilhaft, um das Ausmaß der Bindung der Transglutaminase an den Glutamindonor weiter zu erhöhen. Bevorzugt liegt die Inkubationszeit in einem Bereich von 10 Sekunden bis 5 Minuten, beispielsweise von 0,5 bis 5 Minuten oder von 1 bis 3 Minuten. Die Inkubationszeit kann beispielsweise mindestens 10 Sekunden, mindestens 30 Sekunden oder mindestens 60 Sekunden betragen. Die Inkubationszeit kann beispielsweise höchstens 300 Sekunden oder höchstens 180 Sekunden betragen. Die Inkubationszeit bezeichnet insbesondere den Zeitraum vom Beginn des Inkontaktbringens gemäß Schritt c) bis zum Beginn des Entfernens nicht an den Glutamindonor gebundener Bestandteile der Probe gemäß Schritt d).

Das Inkontaktbringen der Probe mit dem immobilisierten Glutamindonor gemäß Schritt c) des Verfahrens führt insbesondere zu einer Bindung der Transglutaminase an den immobilisierten Glutamindonor.

Glutamin-Reste sind nötig für die Bindung von der Transglutaminase an den immobilisierten Glutamindonor. Transglutaminasen können insbesondere mit einer essentiellen SH-Gruppe an ein Glutamin des Glutamindonors binden, wobei unter Abspaltung von Ammoniak ein Thioester gebildet wird, wie im folgenden Reaktionsschema 1 verdeutlicht.

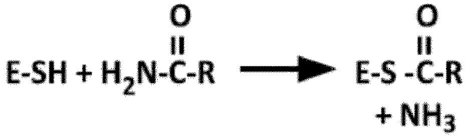

E-SH beschreibt das Enzym (die Transglutaminase) mit seiner essentiellen SH-Gruppe im aktiven Zentrum. Vom Glutamindonor ist die Seitenkette eines Glutamin-Rests gezeigt. Der übrige Glutamindonor ist als "R" dargestellt. Unter Abspaltung von Ammoniak kommt es zur Ausbildung einer Thioesterbindung, die das Enzym E mit dem Glutamindonor R verknüpft.

Diese Reaktion tritt auch physiologisch auf. Transglutaminasen binden jedoch nicht nur an physiologische Glutamindonoren, sondern auch an andere Glutamindonoren, insbesondere solche, die mindestens einen Glutamin-Rest aufweisen.

Die Transglutaminase (insbesondere FXIII) bindet an den immobilisierten Glutamindonor, insbesondere an das immobilisierte Glutamindonorsubstrat. Die Bindung zwischen Transglutaminase und Glutamindonor ist bevorzugt eine kovalente Bindung zwischen einer Aminosäure im Aktiven Zentrum der Glutaminase und einem Glutamin-Rest des Glutamindonors. Die Bindung zwischen Transglutaminase und immobilisiertem Glutamindonor ist insbesondere eine Thioesterbindung. Die Thioesterbindung wird bevorzugt durch Reaktion einer SH-Gruppe der Transglutaminase (insbesondere der essentiellen SH-Gruppe) und einem Glutamin-Rest des immobilisierten Glutamindonors gebildet.

Gemäß Schritt d) des erfindungsgemäßen Verfahrens werden nicht an den immobilisierten Glutamindonor gebundene Bestandteile der Probe entfernt. Ein Bestandteil der Probe, der an den Glutamindonor bindet, ist insbesondere die Transglutaminase, bevorzugt FXIII. Andere Bestandteile der Probe, insbesondere potentiell den Nachweis der Transglutaminase störende Bestandteile, binden bevorzugt nicht oder nur schwach an den Glutamindonor. Eine geringe Bindung ist insbesondere eine solche, die die Entfernung mit einem oder mehreren Waschschritten erlaubt.

Schritt d) des Entfernens nicht an den Glutamindonor gebundener Bestandteile der Probe umfasst bevorzugt einen oder mehrere Waschschritte. Die Anzahl der Waschschritte liegt bevorzugt in einem Bereich von 1 bis 3. Besonders bevorzugt ist das kontinuierliche Waschen, insbesondere durch Überströmen des Trägers des immobilisierten Glutamindonors. Hierbei werden Volumina vom 1- bis 3-fachen des Trägervolumens verwendet. Das Trägervolumen ist das Volumen der im Träger vorhandenen Flüssigkeit.

Die Einzelheiten des Entfernens nicht an den Glutamindonor gebundener Bestandteile der Probe richten sich im Wesentlichen nach der Art der Immobilisierung des Glutamindonors.

Wenn der immobilisierte Glutamindonor als Suspension vorliegt, insbesondere in einem Reaktionsgefäß, kann das Entfernen beispielsweise dadurch erfolgen, dass ein oder mehrere Waschschritte durchgeführt werden, wobei ein Waschschritt die Trennung der Suspension in feste und flüssige Bestandteile, das Entfernen der flüssigen Bestandteile und die Zugabe von Waschlösung, insbesondere Waschpuffer, umfasst. Die Trennung der Suspension in feste und flüssige Bestandteile kann insbesondere mittels Zentrifugation erfolgen. Bevorzugt ist eine Zentrifugation für 0,5 bis 2 Minuten, wobei sich die erforderliche Beschleunigung nach der Art des Trägers richtet.

Wenn das Substrat hingegen immobilisiert auf einem Festkörper, wie beispielsweise dem Papier in einem Teststreifen, vorliegt, kann das Entfernen nicht an den Glutamindonor gebundener Bestandteile der Probe beispielsweise dadurch erfolgen, dass ein oder mehrere Waschschritte durchgeführt werden, wobei ein Waschschritt die Zugabe von Waschlösung zum Festkörper, insbesondere zum Teststreifen, umfasst. Die Waschlösung ist bevorzugt ein Waschpuffer.

Die Waschlösung kann beispielsweise an der Stelle auf den Festkörper aufgebracht werden, an der auch der Glutamindonor immobilisiert ist. Es ist jedoch auch möglich, die Waschlösung an einer Stelle aufzubringen, an der nicht der Glutamindonor immobilisiert ist. Dies kann insbesondere dann eine bevorzugte Möglichkeit sein, wenn der Festkörper, insbesondere der Teststreifen, derart ausgestaltet ist, dass die Waschlösung, beispielsweise durch Kapillarkräfte, durch den Festkörper wandern und so mit dem immobilisierten Glutamindonor in Kontakt gebracht werden kann. Dadurch kann erreicht werden, dass nicht an den Glutamindonor gebundene Bestandteile der Probe entfernt werden.

Die Waschlösung beziehungsweise der Waschpuffer weist bevorzugt eine in etwa isotonische Zusammensetzung auf, z.B. 100 mM NaCl und 50 mM HEPES. Der pH-Wert liegt bevorzugt in einem Bereich von 6,0 bis 9,0, weiter bevorzugt von 7,0 bis 8,0. Der pH-Wert beträgt bevorzugt mindestens 6,0 oder mindestens 7,0. Der pH-Wert beträgt bevorzugt höchstens 9,0 oder höchstens 8,0. Die Waschlösung beziehungsweise der Waschpuffer kann beispielsweise auch PBS (phosphatgepufferte Salzlösung, englisch: "phosphate-buffered saline") sein, insbesondere 137 mM NaCl, 2,7 mM KCl und 12 mM Phosphat (HPO₄²⁻ und H₂PO₄⁻), pH 7,4. Die Waschlösung beziehungsweise der Waschpuffer kann ein oder mehrere Biozide enthalten, beispielsweise NaN₃, bevorzugt in Konzentrationen von 0,01 bis 1,0 mM oder von 0,05 bis 0,5 mM, beispielsweise etwa 0,1 mM.

Physiologisch wird der Thioester zwischen Transglutaminase und Glutamindonor von der ε-Aminogruppe eines Lysins angegriffen und es entsteht unter Aminolyse die Isopeptidbindung Gln-Lys. Die essentielle SH-Gruppe wird wieder freigesetzt. Statt Lysin akzeptiert das Enzym eine ganze Reihe von Amindonoren; auch Alkohole werden eingebaut, jedoch weniger bevorzugt. In Abwesenheit solcher Substrate erfolgt Hydrolyse. Aminolyse und Hydrolyse sind schematisch im folgenden Reaktionsschema 2 gezeigt.

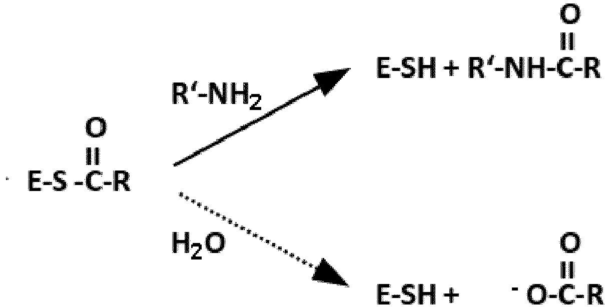

Dabei bezeichnet R'-NH₂ einen Amindonor. Die Hydrolyse verläuft langsamer als die Aminolyse und ist daher gestrichelt dargestellt.

Die Aminolyse kann, wie unten näher beschrieben, zum Nachweis von Transglutaminase verwendet werden. Eine vorzeitige Hydrolyse kann hingegen zu einem verfrühten Freisetzen der Transglutaminase vom immobilisierten Glutamindonor führen, so dass die entsprechende Transglutaminase nicht mehr durch die Aminolyse nachgewiesen werden kann. Für eine möglichst hohe Sensitivität des Nachweises der Transglutaminase sollte also die Hydrolyserate so gering wie möglich sein.

Um eine möglichst stabile Bindung der Transglutaminase an den immobilisierten Glutamindonor zu erhalten, ist es vorteilhaft, wenn der Glutamindonor mehr als einen Glutamin-Rest aufweist. Dadurch kann insbesondere erreicht werden, dass die Transglutaminase auch durch mehrere Waschschritte nicht oder nicht nennenswert vom Glutamindonor entfernt wird. So können mehrere Waschschritte durchgeführt und eine verbesserte Entfernung von nicht an den Glutamindonor gebundenen Bestandteilen der Probe erreicht werden, ohne dadurch den Nachweis der Transglutaminase durch ein übermäßiges Auswaschen zu beeinträchtigen. Eine derart feste Bindung der Transglutaminase an einen Glutamindonor, insbesondere an ein Glutamindonorsubstrat ist überraschend. Ohne an eine bestimmte Theorie gebunden wollen zu sein, könnte der Einfluss der Anzahl der Glutamin-Reste auf die Bindung zumindest teilweise wie folgt zu erklären sein.

Bei einem Glutamindonor mit diversen Glutaminen bestimmt die Umgebung des Glutamins über Aminolyse- und Hydrolysegeschwindigkeit mit. In so einem System wird die Transglutaminase an eins der Glutamine binden. Ist die Hydrolyse schnell genug, wird das Enzym wieder freigesetzt und kann die Reaktion wiederholen. An einem Glutamin mit langsamer Hydrolyse bleibt es immobilisiert. Das System ist selbstoptimierend. Die Transglutaminase bindet an den Glutamin-Rest mit der langsamsten Hydrolyse, entweder direkt oder nach einigen wegen Hydrolyse nicht erfolgreichen Versuchen an anderen Glutamin-Resten. Dies ist im folgenden Reaktionsschema 3 verdeutlicht.

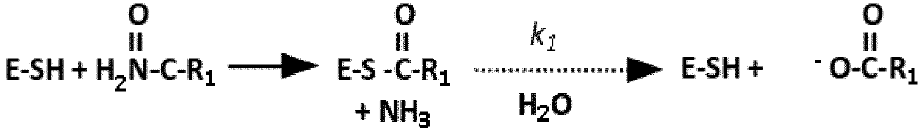

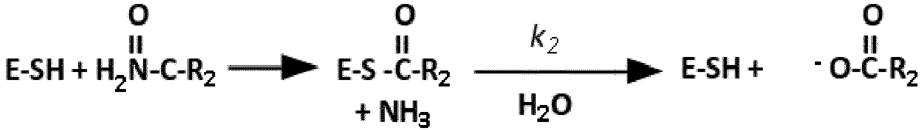

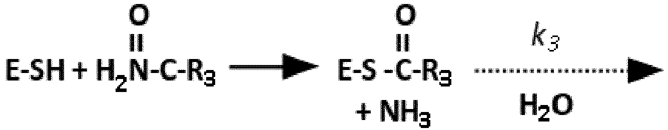

Durch R₁, R₂ und R₃ wird gekennzeichnet, dass es sich um verschiedene Glutamin-Reste des Glutamindonors handelt. Mit k₁, k₂ und k₃ werden die verschiedenen Hydrolysegeschwindigkeitskonstanten bezeichnet. Die Hydrolyserate des dritten Glutamins ist besonders gering und ist daher gestrichelt gezeichnet.

Bevorzugt liegen die Glutamin-Reste im molaren Überschuss gegenüber der Transglutaminase vor.

Gemäß Schritt e) des erfindungsgemäßen Verfahrens wird die an den Glutamindonor gebundene Transglutaminase mit einem Amindonor in Kontakt gebracht, so dass der Amindonor von der Transglutaminase an ein Glutamindonorsubstrat gebunden wird. Dabei wird insbesondere die Bindung zwischen der Transglutaminase und dem immobilisierten Glutamindonor gelöst. Falls der immobilisierte Glutamindonor selbst ein Glutamindonorsubstrat ist, kann die Transglutaminase die Ausbildung einer Bindung zwischen Amindonor und immobilisiertem Glutamindonorsubstrat katalysieren. Ist der immobilisierte Glutamindonor kein Glutamindonorsubstrat, erfolgt die Bindung des Amindonors nicht an den immobilisierten Glutamindonor, sondern an das zusätzlich vorhandene Glutamindonorsubstrat. Das Glutamindonorsubstrat kann insbesondere in solchen Ausführungsformen beispielsweise separat oder gemeinsam mit dem Amindonor hinzugegeben werden.

Der Amindonor kann einen Chromophor beinhalten. Bevorzugt ist insbesondere ein fluoreszenter Amindonor, besonders bevorzugt Dansylcadaverin, Dapoxylcadaverin oder NBD-Cadaverin. "NBD" bezeichnet den Fluorophor 4-Chlor-7-nitro-benzofurazan. Der Amindonor weist bevorzugt mindestens eine fluoreszente Gruppe auf. Der Amindonor kann mindestens einen Farbstoff aufweisen. Ein Amindonor im Sinne der vorliegenden Erfindung ist insbesondere ein Molekül, das mindestens eine primäre Aminogruppe an einer kurzen aliphatischen Kette aufweist. Die primäre Aminogruppe kann beispielsweise die ε-Aminogruppe von Lysin sein oder die freie Aminogruppe eines einseitig substituierten Diamins wie Cadaverin oder Putrescin. Auch eine substituierte Carbohydrazidgruppe kommt in Frage.

Im Reaktionsschema 2 ist ein Amindonor beispielhaft als R'-NH₂ dargestellt.

Insbesondere wird der Amindonor von der an den immobilisierten Glutamindonor gebundenen Transglutaminase an ein Glutamindonorsubstrat gebunden. Dabei löst sich die (Thioester-)Bindung zwischen Transglutaminase und Glutamindonor im Zuge einer Aminolyse. Die Bindung zwischen Amindonor und Glutamindonorsubstrat ist bevorzugt eine Amidbindung, weiter bevorzugt eine Carbonsäureamidbindung.

Der Amindonor ist bevorzugt wasserlöslich. Insbesondere weist der Amindonor bevorzugt eine Wasserlöslichkeit von größer als 10 µM auf. Der Amindonor hat bevorzugt ein Molekulargewicht in einem Bereich von 100 bis 1000 Da, beispielsweise von 150 bis 500 Da. Der Amindonor hat bevorzugt ein Molekulargewicht von mindestens 100 Da oder mindestens 150 Da. Der Amindonor hat bevorzugt ein Molekulargewicht von höchstens 1000 Da oder von höchstens 500 Da.

Das Ausmaß der Bindung des Amindonors an das Glutamindonorsubstrat ist bevorzugt proportional zur Aktivität der an den immobilisierten Glutamindonor gebundenen Transglutaminase. Die Aktivität der an den immobilisierten Glutamindonor gebundenen Transglutaminase ist wiederum bevorzugt proportional zum Anteil der Transglutaminase in der Probe beziehungsweise zur in der Probe vorhandenen Enzymaktivität der Transglutaminase.

Die Einzelheiten des Inkontaktbringens der an den immobilisierten Glutamindonor gebundenen Transglutaminase mit dem Amindonor richten sich im Wesentlichen nach der Art der Immobilisierung des Glutamindonors.

Wenn der immobilisierte Glutamindonor als Suspension vorliegt, insbesondere in einem Reaktionsgefäß, kann das Inkontaktbringen beispielsweise dadurch erfolgen, dass der Amindonor zu der Suspension in das Reaktionsgefäß hinzugegeben wird. Wenn der Glutamindonor hingegen immobilisiert auf einem Festkörper, wie beispielsweise einem Teststreifen, vorliegt, kann das Inkontaktbringen erfolgen, indem der Amindonor und der Festkörper, insbesondere der Teststreifen, in Kontakt gebracht werden. Der Amindonor kann beispielsweise an der Stelle auf den Festkörper aufgebracht werden, an der auch der Glutamindonor immobilisiert ist. Es ist jedoch auch möglich, den Amindonor an einer Stelle aufzubringen, an der nicht der Glutamindonor immobilisiert ist. Dies kann insbesondere dann eine bevorzugte Möglichkeit sein, wenn der Festkörper, insbesondere der Teststreifen, derart ausgestaltet ist, dass der Amindonor, beispielsweise durch Kapillarkräfte, durch den Festkörper wandern und so mit dem immobilisierten Glutamindonor in Kontakt gebracht werden kann.

Der Amindonor liegt vor und/oder beim Inkontaktbringen bevorzugt in Lösung vor. Bevorzugte Pufferlösungen enthalten in etwa isotonische Konzentrationen von NaCl sowie Puffersubstanzen wie Phosphat oder HEPES insbesondere mit einem pH-Wert in einem Bereich von 6,0 bis 9,0, bevorzugt in einem Bereich von 7,0 bis 8,0. Zu kleine und zu große pH-Werte könnten die Hydrolyse des Thioesters begünstigen und damit das Signal verschlechtern. Der pH-Wert beträgt bevorzugt mindestens 6,0 oder mindestens 7,0. Der pH-Wert beträgt bevorzugt höchstens 9,0 oder höchstens 8,0. Die Pufferlösung kann ein oder mehrere Biozide enthalten, beispielsweise NaN₃, bevorzugt in Konzentrationen von 0,01 bis 1,0 mM oder von 0,05 bis 0,5 mM, beispielsweise etwa 0,1 mM.

Gemäß Schritt f) des erfindungsgemäßen Verfahrens wird die enzymatischen Aktivität der Transglutaminase anhand eines Signals bestimmt, das durch die Bindung des Amindonors an das Glutamindonorsubstrat erzeugt wird. Das Signal kann insbesondere der Geschwindigkeit der durch die Transglutaminase katalysierten Bindung des Amindonors an das Glutamindonorsubstrat proportional sein. Das Signal kann also proportional zur zeitlichen Zunahme des an das Glutamindonorsubstrat gebundenen Amindonors sein. In solchen Ausführungsformen gilt: Je größer die enzymatische Aktivität der Transglutaminase in der Probe ist, desto größer ist die Geschwindigkeit der Bindung des Amindonors an das Glutamindonorsubstrat und entsprechend das Signal. Das Signal kann insbesondere eine spektrale Änderung je Zeiteinheit sein.

Um das Hintergrundsignal zu reduzieren, können vor der Bestimmung der Menge des gebundenen Amindonors ein oder mehrere Waschschritte durchgeführt werden. Insbesondere können ein oder mehrere Waschschritte so durchgeführt werden, wie hinsichtlich der Entfernung nicht an das Substrat gebundener Bestandteile der Probe gemäß Schritt d) beschrieben. Die Anzahl der Waschschritte liegt bevorzugt in einem Bereich von 1 bis 3 oder es wird ein kontinuierliches Überströmen mit Waschpuffer verwendet. Mit den Waschschritten kann insbesondere nicht an das Glutamindonorsubstrat gebundener Amindonor entfernt werden.

Bevorzugt wird das Signal optisch bestimmt. Beispielsweise kann das Signal mit Hilfe von Photometrie bestimmt werden. Das Signal kann insbesondere mittels Fluorimetrie bestimmt werden.

Die Einzelheiten der Bestimmung des Signals richten sich insbesondere nach dem verwendeten Fluorophor. Einige Fluorophore, beispielsweise Dansyl, ändern beispielsweise ihr Absorptionsmaximum beim Einbau in das Glutamindonorsubstrat (Protein), so dass auch ein Intensitätsverhältnis Emission(eingebaut)/Emission(nicht eingebaut) beziehungsweise dessen zeitliche Änderung gemessen werden kann. Insbesondere in solchen Ausführungsformen kann auf die Durchführung eines oder mehrerer Waschschritte verzichtet werden.

Die Zeit vom Inkontaktbringen der Probe mit dem immobilisierten Glutamindonor bis zur Bestimmung der enzymatischen Aktivität der Transglutaminase liegt bevorzugt in einem Bereich von 1 bis 10 Minuten, beispielsweise von 2 bis 5 Minuten. Die Zeit kann beispielsweise mindestens 1 Minuten oder mindestens 2 Minuten betragen. Die Zeit kann beispielsweise höchstens 5 Minuten oder höchstens 10 Minuten betragen.

Besonders bevorzugt umfasst das erfindungsgemäße Verfahren die folgenden Schritte:
a) Bereitstellen eines immobilisierten FXIII-Substrats, wobei das FXIII-Substrat ein Peptid ist, insbesondere ein Polypeptid oder Protein, und wobei das FXIII-Substrat mindestens einen Glutamin-Rest umfasst,
b) Aktivierung des in der Probe vorhandenen FXIII,
c) Inkontaktbringen der Probe mit dem FXIII-Substrat, so dass in der Probe vorhandener FXIII an das FXIII-Substrat bindet,
d) Entfernung nicht an das FXIII-Substrat gebundener Bestandteile der Probe,
e) Inkontaktbringen des an das FXIII-Substrat gebundenen FXIII mit einem Amindonor, so dass der Amindonor von FXIII an das FXIII-Substrat gebunden wird, und
f) Bestimmung der enzymatischen Aktivität von FXIII anhand eines Signals, das durch die Bindung des Amindonors an das FXIII-Substrat erzeugt wird.

Insbesondere kann das Signal der Geschwindigkeit der durch FXIII katalysierten Bindung des Amindonors an das FXIII-Substrat proportional sein.

Die vorliegende Erfindung betrifft auch eine Vorrichtung zur Bestimmung der enzymatischen Aktivität von Transglutaminasen, beispielsweise von Blutgerinnungsfaktor XIII, insbesondere einen Teststreifen, beispielsweise in Form eines Lateral Flow Tests. Die Vorrichtung ist bevorzugt eine Vorrichtung zur Durchführung des erfindungsgemäßen Verfahrens.

Die Vorrichtung umfasst mindestens eine Reaktionszone, bevorzugt genau eine Reaktionszone.

Die Vorrichtung weist bevorzugt drei oder mehr Zonen auf, insbesondere drei Zonen. Bevorzugt umfasst die Vorrichtung mindestens eine Auftragszone, mindestens eine Reaktionszone und mindestens eine Saugzone. Die Vorrichtung kann einen Träger (beispielsweise aus einem Trägermaterial) umfassen, auf dem die Materialien der verschiedenen Zonen aufgebracht sind. Das Vorhandensein eines Trägers ist optional. In einigen Ausführungsformen der Erfindung weist die Vorrichtung keinen Träger auf.

Bevorzugt enthält die Reaktionszone einen immobilisierten Glutamindonor, insbesondere in einer Menge von 0,01 bis 10 mg, beispielsweise von 0,05 bis 5 mg oder von 0,1 bis 2 mg. Die Menge kann beispielsweise mindestens 0,01 mg, mindestens 0,05 mg oder mindestens 0,1 mg betragen. Die Menge kann beispielsweise höchstens 10 mg, höchstens 5 mg oder höchstens 2 mg betragen.

Der immobilisierte Glutamindonor ist insbesondere ein Glutamindonorsubstrat, bevorzugt ein FXIII-Substrat. Der immobilisierte Glutamindonor ist insbesondere ein Protein. Bevorzugt sind Proteine mit einem Molgewicht von 10 bis 300 kDa und einem Glutamingehalt von 3 bis 15%. Bevorzugt sind Proteine mit einem Molgewicht von 10 bis 300 kDa, beispielsweise von 10 bis 100 kDa oder von 12 bis 30 kDa. Das Molgewicht des Glutamindonors kann beispielsweise mindestens 10 kDa, oder mindestens 12 kDa betragen. Das Molgewicht des Glutamindonors kann beispielsweise höchstens 300 kDa, höchstens 100 kDa oder höchstens 30 kDa betragen. Bevorzugt liegt der Glutamingehalt des Glutamindonors in einem Bereich von 3% bis 15% bezogen auf die Gesamtanzahl an Resten, beispielsweise in einem Bereich von 5% bis 12% oder von 7% bis 10%. Der Glutamingehalt des Glutamindonors kann beispielsweise mindestens 3%, mindestens 5% oder mindestens 7% betragen. Der Glutamingehalt des Glutamindonors kann beispielsweise höchstens 15%, höchstens 12% oder höchstens 10% betragen.

Die Immobilisierung des Glutamindonors umfasst bevorzugt eine oder mehrere der folgenden Maßnahmen:
- Fällung und Adsorption
- Ionische Bindung an DEAE-funktionalisierte Oberflächen
- Kovalente Bindung durch Formaldehyd, Glutardialdehyd
- Kovalente Bindung unter Verwendung von EDC/NHS bei Amin- oder COOHfunktionalisierter Trägeroberfläche
- Kovalente Bindung durch Dimethylsuberimidat, Dimethylpimelimidat, Dimethyladipimidat bei aminfunktionalisierter Trägeroberfläche
- Kovalente Bindung durch trägergebundene Epoxide, Isocyanate
- Kovalente Bindung über Aminogruppen an BrCN-aktivierte Polysaccharide.

Der immobilisierte Glutamindonor kann Modifikationen, beispielsweise chemische Modifikationen aufweisen. Besonders bevorzugt sind Modifikationen von Lysin-Resten. Insbesondere kann der Glutamindonor methyliert, acetyliert oder succinyliert sein, beispielsweise dimethyliert, insbesondere N,N-dimethyliert oder auch acetamidiniert. Besonders bevorzugt ist der Glutamindonor ausgewählt aus der Gruppe bestehend aus Casein und Lysozym, insbesondere Dimethylcasein und succinyliertes Lysozym.

Die Reaktionszone ist die Zone der Vorrichtung, in der die Bindung der Transglutaminase an den immobilisierten Glutamindonor stattfindet (insbesondere gemäß Schritt c) des Verfahrens) und in der die Bindung des Amindonors an das Glutamindonorsubstrat stattfindet (insbesondere gemäß Schritt e) des Verfahrens). Die Vorrichtung weist bevorzugt genau eine Reaktionszone auf. Als Alternative können auch zwei oder mehr Reaktionszonen vorgesehen sein.

Die Auftragszone, die Reaktionszone und die Saugzone können dieselben Materialien aufweisen oder aus denselben Materialien bestehen. Sie können aber auch unterschiedliche Materialien aufweisen oder aus unterschiedlichen Materialen bestehen. Infrage kommen insbesondere sämtliche poröse Materialien, die einen kapillargetriebenen Transport von Flüssigkeiten und darin gelöster oder suspendierter Stoffen erlauben. Beispiele für solche Materialien sind etwa Nitrocellulosemembranen, Glasfaservließe, Papiere, Filter aus Papier oder hydrophilisierten Polymerfasern (z.B. Polyester, Polypropylen) sowie sämtliche Komposite aus den vorgenannten Materialien. Die Materialien können dabei beispielsweise aus gewebten oder ungewebten Fasern oder Fäden, aber auch aus offenen oder geschlossenen Schäumen bestehen.

Bevorzugt weist die Vorrichtung folgende Materialien auf oder besteht daraus:
ungewebte Vliese aus Papier-, Polymer- oder Glasfasern
Die Vorrichtung kann eine Einhausung aufweisen, beispielsweise eine Einhausung aus Kunststoff.

Zusätzlich zu den Materialien der Auftragszone, der Reaktionszone und der Saugzone kann die Vorrichtung ein oder mehrere weitere Materialien umfassen, beispielsweise ein Trägermaterial. Das Trägermaterial kann beispielsweise aus Glas, Plexiglas, einer Polymerfolie oder auch aus einer Beschichtung bestehen, welche eine Barriere für die in der Vorrichtung verwendete Flüssigkeit darstellt. Ein solches Trägermaterial ist aber nicht zwingend nötig, da Luft, die die Vorrichtung umgibt, bereits eine hinreichende (und hydrophobe) Barriere darstellt.

Bevorzugt weist die Auftragszone folgende Materialien auf oder besteht daraus:
ungewebte Vliese aus Papier-, Polymer- oder Glasfasern
Bevorzugt weist die Reaktionszone folgende Materialien auf oder besteht daraus:
   ungewebte Vliese aus Papier-, Polymer- oder Glasfasern
   Bevorzugt weist die Saugzone folgende Materialien auf oder besteht daraus: Papier oder Faservlies mit Aufnahmevermögen, das über dem der Auftrags- und Reaktionszone liegt. Dies kann sowohl durch eine höhere Dicke, Länge oder Breite des Fasermaterials der Saugzone erreicht werden. Ausschlaggebend ist das Aufnahmevermögen, nicht die Geometrie.

Bevorzugt ist die Dicke oder Länge der Saugzone mindestens doppelt so groß wie die Dicke oder Länge der Auftragszone oder wie die Dicke oder Länge der Reaktionszone. Auftragszone und Reaktionszone haben bevorzugt dieselbe oder im Wesentlichen dieselbe Dicke. Bevorzugt liegt die Dicke der Saugzone in einem Bereich vom 2-Fachen der Dicke der Auftragszone oder der Reaktionszone bis zum 10-Fachen der Dicke der Auftragszone oder der Reaktionszone.

Bevorzugt ist das Aufnahmevermögen der Saugzone mindestens doppelt so groß wie das Aufnahmevermögen der Auftragszone oder wie das Aufnahmevermögen der Reaktionszone. Auftragszone und Reaktionszone haben bevorzugt dasselbe oder im Wesentlichen dasselbe Aufnahmevermögen. Bevorzugt liegt das Aufnahmevermögen der Saugzone in einem Bereich vom 2-Fachen des Aufnahmevermögens der Auftragszone oder der Reaktionszone bis zum 10-Fachen des Aufnahmevermögens der Auftragszone oder der Reaktionszone.

Die Vorrichtung kann Auftragszone und Reaktionszone in einer kombinierten Auftrags-Reaktionszone enthalten. Bevorzugt befinden sich Auftragszone und Reaktionszone jedoch an räumlich verschiedenen Positionen der Vorrichtung. Dies bedeutet allerdings nicht, dass es keine Verbindung zwischen Auftragszone und Reaktionszone gibt. Ganz im Gegenteil umfasst die Vorrichtung mindestens eine Verbindung, bevorzugt genau eine Verbindung zwischen Auftragszone und Reaktionszone.

Die Auftragszone ist die Zone der Vorrichtung, in der bevorzugt die Probe und/oder der Amindonor und/oder Waschlösung aufgebracht wird. Es ist möglich, verschiedene Auftragszonen vorzusehen, beispielsweise mindestens eine Auftragszone zum Aufbringen der Probe, mindestens eine Auftragszone zum Aufbringen des Amindonors und/oder mindestens eine Auftragszone zum Aufbringen der Waschlösung. Bevorzugt ist die Auftragszone der Vorrichtung jedoch eine kombinierte Auftragszone, in der die Probe, der Amindonor und die Waschlösung aufgebracht werden können. Bevorzugt enthält die Vorrichtung genau eine Auftragszone.

Die Verbindung von Auftragszone und Reaktionszone ermöglicht es, dass eine in der Auftragszone aufgebrachte Probe und/oder ein in der Auftragszone aufgebrachter Amindonor und/oder in der Auftragszone aufgebrachte Waschlösung von der Auftragszone zur Reaktionszone gelangt. Der Transport aus der Auftragszone in die Reaktionszone kann insbesondere mittels Kapillarkräften geschehen.

Bevorzugt enthält die Vorrichtung genau eine Saugzone. Alternativ können auch zwei oder mehr Saugzonen vorgesehen sein. Bevorzugt enthält die Vorrichtung mindestens eine Verbindung, bevorzugt genau eine Verbindung zwischen der Reaktionszone und der Saugzone. Die Saugzone dient insbesondere dazu, nicht an den immobilisierten Glutamindonor gebundene Reagenzien, wie beispielsweise nicht gebundenen Amindonor, und nicht an den immobilisierten Glutamindonor gebundene Probenbestandteile (insbesondere Blutbestandteile) aus der Reaktionszone zu entfernen. Auch überschüssige Waschlösung oder sonstige Lösungen können mit Hilfe der Saugzone aus der Reaktionszone entfernt werden. Der Transport aus der Reaktionszone in die Saugzone kann insbesondere mittels Kapillarkräften geschehen.

Die Vorrichtung enthält bevorzugt genau zwei Verbindungen, nämlich eine Verbindung zwischen der Auftragszone und der Reaktionszone sowie eine Verbindung zwischen der Reaktionszone und der Saugzone. Die Verbindungen sind kapillaraktiv, sodass ein ununterbrochener Flüssigkeitstransport gewährleistet ist. Wenn die Zonen aus unterschiedlichen Materialien gefertigt sind, kann die Verbindung zwischen den Zonen aus dem Material einer der beiden Zonen oder aus einer Mischung der Materialien bestehen oder diese umfassen. Es ist auch möglich, dass das Material der Verbindung ein weiteres Material umfasst oder daraus besteht, das nicht Teil einer der beiden verbundenen Zonen ist. Sind die Zonen so gestaltet, dass kein Materialwechsel erfolgt, so sind die Verbindungen bevorzugt aus demselben Material. Zum Beispiel kann die gesamte Vorrichtung auch aus einem Faservlies, z.B. Papier, gefertigt sein.

### Beschreibung der Figuren

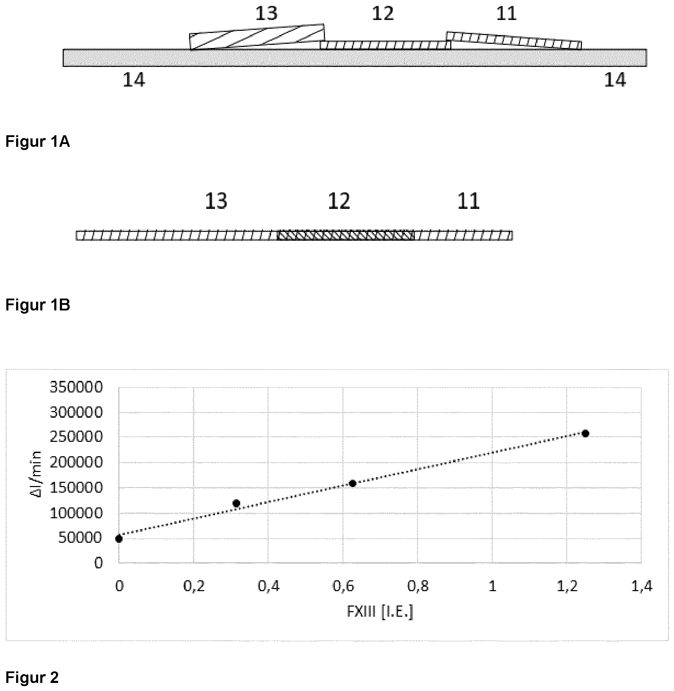

Figur 1A zeigt einen schematischen Aufbau einer erfindungsgemäßen Vorrichtung in Form eines Lateral Flow Tests. Die Vorrichtung enthält eine Auftragszone 11, eine Reaktionszone 12 und eine Saugzone 13. Die Auftragszone 11, die Reaktionszone 12 und die Saugzone 13 befinden sich auf einem Träger 14. Die Dicke der Saugzone 13 ist größer als die Dicke der Auftragszone 11 und der Reaktionszone 12, die dieselbe Dicke aufweisen. Es ist beispielsweise möglich, als Material für die Auftragszone 11, die Reaktionszone 12 und die Saugzone 13 jeweils Papier zu verwenden. Die drei Papiere können mit leichter Überlappung auf den Träger 14 (beispielsweise einen Objektträger, bevorzugt aus Plexiglas) aufgelegt werden. Im Gegensatz zur Schemazeichnung legen sich die Papiere in der Praxis in der Regel flexibel an die Oberfläche des Trägers 14 an. Alternativ können die Zonen 11, 12 und 13 auch in einem kontinuierlichen Faservlies, z.B. einem Papier, realisiert werden. Dieser Aufbau ist in Figur 1B dargestellt. Ein Träger ist optional und in Figur 1B nicht gezeigt.

Figur 2 zeigt die Abhängigkeit des Signals von der Menge an FXIII. Mit einer wie in Figur 1A gezeigten Vorrichtung wurde der Amindonor Dansylcadaverin von der Transglutaminase FXIII an ein immobilisiertes FXIII-Substrat gebunden. Durch die Bindung von Dansylcadaverin an das immobilisierte FXIII-Substrat kommt es zu einer Änderung spektralen Änderung der Fluoreszenz des Dansylcadaverins, die mittels eines Imagers aufgezeichnet wurde. Die Fluoreszenzzunahme wurde bei einer Anregungswellenlänge von 365 nm und einer Emissionswellenlänge von 505-565 nm gemessen. In Figur 2 ist auf der x-Achse die Menge der Transglutaminase (in Internationalen Einheiten [I.E.]) aufgetragen. Auf der y-Achse ist das Signal als Intensitätsänderung ΔI pro Minute aufgetragen. Die Intensitätsänderung ist die Differenz der Fluoreszenzintensität 30 Minuten nach Zugabe des Amindonors und der Fluoreszenzintensität 5 Minuten nach Zugabe des Amindonors. Die erhaltene Fluoreszenzintensitätsänderung wurde durch den Zeitraum von 25 Minuten dividiert, um das Signal als Intensitätsänderung ΔI pro Minute zu erhalten. Es ergibt sich ein linearer Zusammenhang zwischen der Menge der Transglutaminase (in Internationalen Einheiten [I.E.]) und dem Signal (Intensitätsänderung ΔI pro Minute).

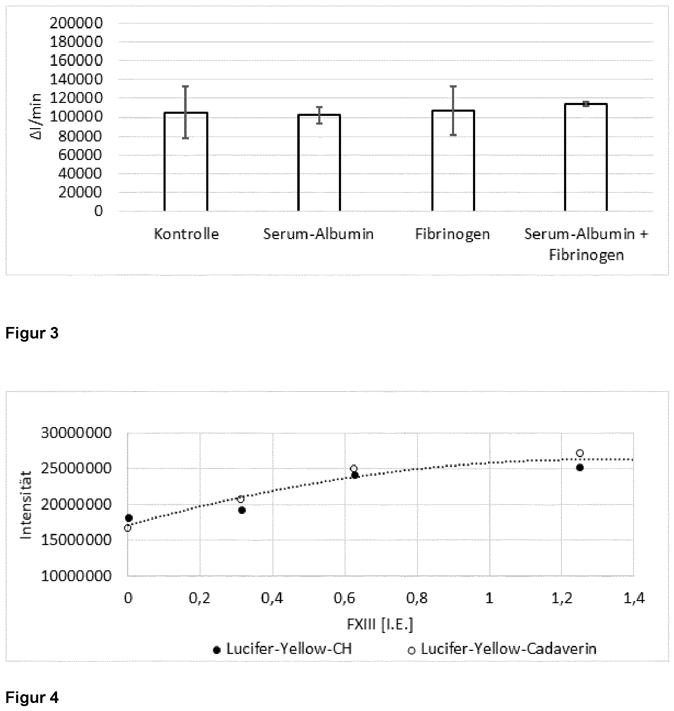

Figur 3 zeigt ein Balkendiagramm des Signals (Intensitätsänderung ΔI pro Minute) ± Standardabweichung (n = 3 unabhängige Experimente). Untersucht wurde der Einfluss potentieller Störkomponenten im Vergleich zu einer Kontrolle ohne diese Störkomponenten. Als Störkomponenten wurden Serum-Albumin, Fibrinogen sowie eine Kombination von SerumAlbumin und Fibrinogen getestet. Eine signifikante Änderung des Signals durch die potentiellen Störkomponenten wurde nicht festgestellt. Die Messung ist also unbeeinflusst durch die Gegenwart der entsprechenden Komponenten.

Figur 4 zeigt die Abhängigkeit des Signals von der Menge an FXIII. Mit einer wie in Figur 1A gezeigten Vorrichtung wurden der Amindonor Lucifer-Yellow-Cadaverin oder der Amindonor Lucifer-Yellow-CH von der Transglutaminase FXIII an ein immobilisiertes FXIII-Substrat gebunden. Lucifer-Yellow-CH besitzt statt des Cadaverin-Rests eine Carbohydrazid-Kette. Die Fluoreszenzzunahme wurde bei einer Anregungswellenlänge von 460 nm ±20 nm und einer Emissionswellenlänge von 505-565 nm gemessen. In Figur 4 ist auf der x-Achse die Menge der Transglutaminase (in Internationalen Einheiten [I.E.]) aufgetragen. Auf der y-Achse ist die Intensität aufgetragen. Es ergibt sich ein klarer Zusammenhang zwischen der Menge der Transglutaminase (in Internationalen Einheiten [I.E.]) und der gemessenen Fluoreszenzintensität.

### Beispiele

### Material und Methoden für sämtliche Beispiele (falls zutreffend)

### Geräte

UV-Lampe 365 nm: neolab
Fotoapparat: P 40 Lite (Huawei)
Imager (unter anderem zur Anregung bei 460 nm): Fusion FX 7 Edge (Vilber)
Puffer und Papiere
Standardpuffer: 50 mM HEPES, 100 mM NaCl, 0,1 mM NaN₃, pH 7,4
Reaktionslösung zur DansCad-Anfärbung von DEAE-Cellulose/Dimethylcasein: Standardpuffer mit Dansyl-Cadaverin (50 µM), DTT (2 mM) und CaCl₂ (5mM)
Reaktionslösung für LFT (Lateral Flow Test): Wie für Cellulose-Anfärbung, aber 350 µM Dansylcadaverin
Papier für Auftrags- und Reaktionszone: Whatman 1 CHR (Chromatographie)
Saugpapier: Whatman gel blot paper
Enzyme
FXIII: Fibrogammin (CSL Behring). Die Menge wird in Internationalen Einheiten [I.E.] angegeben. Diese Einheiten werden vom Hersteller durch Vergleich mit einem standardisierten Normalplasma ermittelt.

### Beispiel 1 (Bindung von FXIII an Dimethylcasein adsorbiert an DEAE-Cellulose)

0,625 I.E. FXIIIₐ wurden 10 min mit einem immobilisierten Glutamindonorsubstrat (1,25 mg Dimethylcasein an 125 mg DEAE-Cellulose) inkubiert. Dann wurde 0 bis 4 Mal mit Standardpuffer pH 7,4 gewaschen. Anschließend wurde 50 µM Dansylcadaverin als Amindonor zugegeben. Unter UV-Beleuchtung (365 nm) wurde die Fluoreszenz des an Dimethylcasein gebundenen Dansylcadaverin detektiert.

Überraschenderweise wurde gefunden, dass FXIIIₐ an das immobilisierte Glutamin-Donor-Protein genügend fest bindet, um aus einer Probe extrahiert und angereichert zu werden.

Ein oder zweimaliges Waschen vermindert die Fluoreszenz nicht sichtbar. Es wird also ein großer Teil des zugesetzten FXIII immobilisiert. Auch nach mehrfachem Waschen lässt sich FXIII noch sehr gut nachweisen.

### Beispiel 2 (Reversible Bindung von FXIII an Dimethylcasein-DEAE-Cellulose)

Gegenüber Beispiel 1 wurden nur die Waschschritte geändert.
Beispiel 2A: Reaktion mit Dansylcadaverin nach dreimaligem Waschen mit Standardpuffer
Beispiel 2B: Reaktion nach zweimaligem Waschen mit 0,1 M Glycinethylester zur Aminolyse des Intermediats, einmal mit Puffer
Beispiel 2C: Zweimaliges Waschen mit 0,1 M Glycinethylester + 10 µM Silbernitrat zum Blockieren von reaktiven Thiolen, einmal mit Puffer

Die Bindung von FXIII an Dimethylcasein konnte durch Amindonoren gelöst werden. Eine Blockade der essentiellen -SH-Gruppe durch Ag⁺-lonen war nicht nötig; in Gegenwart des Amindonors blieb FXIII in Lösung. Sowohl die Bindung als auch die Anfärbung beruhen auf der spezifischen Aktivität des Analyten FXIII.

### Beispiel 3 (Nachweis von FXIII auf dem LFT im Dansylcadaverintest)

Die Beispiele 1 und 2 wurden in Reaktionsgefäßen (Eppendorf-Tubes) durchgeführt. Im Gegensatz dazu wurde das Beispiel 3 an einer Vorrichtung mit einer Auftragszone, einer Reaktionszone und einer Saugzone durchgeführt. Zum Nachweis von FXIII eignete sich ein Papier-basierter, ganz einfacher manuell gefertigter Lateral Flow Test (LFT).

Auf die Auftragszone wurden nacheinander Analyt-, Spül- und Reaktionslösung aufgegeben. 50 µl Analyt wurden innerhalb von ca. 20 Sekunden aufgesaugt; danach folgten 100 µL Waschpuffer, um den Analyten in die Reaktionszone zu transportieren. Nach 10minütiger Inkubation zur Bindung von FXIII an das Substrat folgten 100 µL Reaktionslösung zum Auswaschen nicht gebundener Komponenten.
- Analyt: 0 / 0,313 / 0,625 / 1,25 I.E. FXIII in Standardpuffer mit 200 mM CaCl₂.
- Waschpuffer: Standardpuffer mit 200 mM CaCl₂
- Reaktionslösung : 350 µM Dansylcadaverin, 2 mM Dithiothreitol in Standardpuffer

Die Zunahme der Fluoreszenz wurde im Imager bei Raumtemperatur aufgezeichnet. Zwischen 5 und 30 Minuten ergab sich die in der Abbildung gezeigte Abhängigkeit. Die Ergebnisse sind in Figur 2 gezeigt.

Da es sich um eine im wesentlichen lineare Zunahme der Intensität handelt, sind auch Messungen nach kürzeren Zeiten möglich.

Wurde statt der FXIII-Lösung eine stark fluoreszierende Mischung von Serumalbumin (50 mg/ml) und Dansylcadaverin (350 µM) in Standardpuffer verwendet, dann wurde nach Waschen mit Standardpuffer inklusive 50 µM Dansylcadaverin in der Reaktionszone nur die schwache Fluoreszenz des ungebundenen Dansylcadaverins beobachtet. Die starke Fluoreszenz des Albumin-gebundenen Dansylcadaverins war in der Saugzone sichtbar. Das Protein wurde also aus der Reaktionszone ausgewaschen. Die in Figur 2 gezeigte FXIII-spezifische Reaktion beruhte also auf der Bindung und Retention des Analyten FXIII am Ca²⁺- gefällten Dimethylcasein.

### Beispiel 4 (Reaktion in Gegenwart potentieller Störkomponenten)

Der Testaufbau und die Bedingungen waren die gleichen wie im Beispiel 3.

Die Bindung von FXIII und die Anfärbung mit Dansylcadaverin waren unabhängig von zugesetztem Fibrinogen (5 mg/ml) oder Serumalbumin (50 mg/ml) auch in den hohen physiologisch vorliegenden Konzentrationen (siehe Figur 3). Die Bevorzugung des nichtphysiologischen Substrates war überraschend. Eine Entfernung von Fibrinogen ist hier völlig unnötig.

### Beispiel 5 (FXIII-Abhängigkeit beim Einbau von Lucifer-Yellow)

Es ist bekannt, dass auch andere Fluorophore in Abhängigkeit von der Polarität der Umgebung ihre Fluoreszenzintensität ändern, z.B. Dapoxyl-, Aminonaphthalimid- oder NBD-Fluorophore. Da die Umsetzung mit FXIII unabhängig vom Chromophor mit vielen Aminen gelingt, kann man durch Wahl des Fluorophors die Wellenlängen für Anregung und Emission entsprechend auswählen.

Auch ohne diese Fluoreszenzänderung lässt sich FXIII nachweisen. Geeignet sind z.B. Lucifer-Yellow CH und Lucifer-Yellow-Cadaverin. Die Reaktion wurde im Lateral Flow Aufbau analog zum Verfahren mit Dansylcadaverin durchgeführt (siehe Beispiel 3). Da aber die Fluoreszenz des Lucifer-Yellow-Farbstoffs sich beim Einbau nicht wesentlich ändert, wurde nach 20 Minuten Einwirkzeit zweimal mit Standardpuffer +200 mM CaCl₂ gewaschen und danach die Fluoreszenz am Imager gemessen. Die Ergebnisse sind in Figur 4 gezeigt.

Der Nachweis funktioniert demnach unabhängig vom Chromophor. Auch das Carbohydrazid im Lucifer YellowCH wird ähnlich wie der Cadaverinrest als Amindonor akzeptiert. Vorteilhaft bei Lucifer Yellow ist die gegenüber Dansylcadaverin längere Anregungswellenlänge. Wegen fehlender Polaritätssensitivität sind aber zusätzliche Waschschritte zur Entfernung des unreagierten Farbstoffs erforderlich. Messungen per Remissionsphotometrie sind ebenfalls möglich. Hierfür kann man bei Bedarf Farbstoffe mit hohen Extinktionskoeffizienten bis hin zu Cadaverin-modifizierten Nanogold-Partikeln einsetzen.

### Bezuqszeichenliste

- 11: Auftragszone
- 12: Reaktionszone
- 13: Saugzone
- 14: Träger

## Patentansprüche

1. Verfahren zur Bestimmung der enzymatischen Aktivität einer Transglutaminase in einer Probe, wobei das Verfahren die folgenden Schritte umfasst:
a) Bereitstellen eines immobilisierten Glutamindonors, wobei der Glutamindonor ein Peptid ist, insbesondere ein Polypeptid oder Protein, und wobei der Glutamindonor mindestens einen Glutamin-Rest umfasst,
b) Aktivierung der in der Probe vorhandenen Transglutaminase,
c) Inkontaktbringen der Probe mit dem Glutamindonor, so dass in der Probe vorhandene Transglutaminase an den Glutamindonor bindet,
d) Entfernung nicht an den Glutamindonor gebundener Bestandteile der Probe,
e) Inkontaktbringen der an den Glutamindonor gebundenen Transglutaminase mit einem Amindonor, so dass der Amindonor von der Transglutaminase an ein Glutamindonorsubstrat gebunden wird, und
f) Bestimmung der enzymatischen Aktivität der Transglutaminase anhand eines Signals, das durch die Bindung des Amindonors an das Glutamindonorsubstrat erzeugt wird.

2. Verfahren nach Anspruch 1, wobei die Probe eine Blutprobe ist.

3. Verfahren nach mindestens einem der vorhergehenden Ansprüche, wobei der Glutamindonor an einen Träger gebunden ist.

4. Verfahren nach mindestens einem der vorhergehenden Ansprüche, wobei Schritt c) einen Inkubationsschritt umfasst und wobei die Inkubationszeit in einem Bereich von 10 Sekunden bis 5 Minuten liegt.

5. Verfahren nach mindestens einem der vorhergehenden Ansprüche, wobei der Glutamindonor das Glutamindonorsubstrat ist.

6. Verfahren nach mindestens einem der vorhergehenden Ansprüche, wobei die Bindung zwischen Transglutaminase und Glutamindonor eine kovalente Bindung zwischen einer Aminosäure im Aktiven Zentrum der Glutaminase und einem Glutamin-Rest des Glutamindonors ist.

7. Verfahren nach mindestens einem der vorhergehenden Ansprüche, wobei die Bindung zwischen Transglutaminase und immobilisiertem Glutamindonor eine Thioesterbindung ist.

8. Verfahren nach mindestens einem der vorhergehenden Ansprüche, wobei Schritt d) einen oder mehrere Waschschritte umfasst.

9. Verfahren nach mindestens einem der vorhergehenden Ansprüche, wobei der Amindonor mindestens eine fluoreszente Gruppe oder mindestens einen Farbstoff aufweist.

10. Verfahren nach mindestens einem der vorhergehenden Ansprüche, wobei die Transglutaminase Blutgerinnungsfaktor XIII ist.

11. Verfahren nach mindestens einem der vorhergehenden Ansprüche, wobei das Signal optisch bestimmt wird.

12. Verfahren nach mindestens einem der vorhergehenden Ansprüche, wobei der Amindonor Dansylcadaverin, Dapoxylcadaverin oder NBD-Cadaverin ist.

13. Vorrichtung, insbesondere zur Durchführung des Verfahrens nach mindestens einem der Ansprüche 1 bis 12, zur Bestimmung der enzymatischen Aktivität einer Transglutaminase in einer Probe, wobei die Vorrichtung mindestens eine Reaktionszone umfasst und wobei die Reaktionszone immobilisierten Glutamindonor umfasst, insbesondere in einer Menge von 0,01 bis 10 mg.

14. Vorrichtung nach Anspruch 13, wobei der Glutamindonor ein Protein mit einem Molgewicht von 10 bis 300 kDa ist.

15. Vorrichtung nach mindestens einem der Ansprüche 13 bis 14, wobei die Vorrichtung mindestens eine Auftragszone umfasst und wobei die Vorrichtung derart eingerichtet ist, dass in der Auftragszone aufgebrachte Probe mittels Kapillarkräften in die Reaktionszone gelangen kann.
